# EUROPEAN PATENT APPLICATION

(11) **EP 3 778 851 A1**
(43) Date of publication of application: **17.02.2021**
(21) Application number: 19774406.3
(22) Date of filing: 29.03.2019
(51) Int. Cl.: C12M 1/00, C12Q 1/6844

(54) **FULLY AUTOMATIC GENETIC TESTING DEVICE**

(30) Priority: 29.03.2018 US 201815940235
(71) Applicant: EIKEN KAGAKU KABUSHIKI KAISHA, Tokyo 110-8408 (JP)
(72) Inventor: SEGAWA, Yuji, Shimotsuga-gun, Tochigi 329-0114 (JP); WATANABE, Hidetoshi, Shimotsuga-gun, Tochigi 329-0114 (JP); YONEKAWA, Toshihiro, Shimotsuga-gun, Tochigi 329-0114 (JP); KAZUMI, Fumihiko, Tokyo 110-8408 (JP); PETERSON, Rod, Irvine, California 92618 (US); MOSKALEV, Anatoly, Irvine, California 92618 (US); FORSHAGER, Goran, Irvine, California 92618 (US); FOSTER, James, Irvine, California 92618 (US)
(74) Representative: Grünecker Patent- und Rechtsanwälte PartG mbB
(86) International application number: PCT/JP2019/013985
(87) International publication number: WO 2019/189753

(57) **Abstract**

The present invention aims to provide a compact, fully automatic genetic testing device with which a step of extracting and purifying a nucleic acid from a sample, a step of injecting a solution containing the nucleic acid, and a step of amplifying and detecting the nucleic acid, can be fully automatically carried out, and which enables a physician to carry out genetic testing at a position close to a patient. The fully automatic genetic testing device comprises: a first unit for extracting and purifying a nucleic acid from a sample; a second unit for injecting a solution containing the nucleic acid to a testing chip having reaction sites for amplification of the nucleic acid; and a third unit for optically detecting the nucleic acid amplified in the reaction sites.

## Description

### TECHNICAL FIELD

The present invention relates to a fully automatic genetic testing device. More specifically, the present invention relates to a compact, fully automatic genetic testing device with which a step of extracting and purifying nucleic acid contained in a sample, a step of injecting a solution containing the nucleic acid to a test chip, and a step of amplifying and detecting the nucleic acid can be fully automatically carried out, and with which genetic testing can be carried out near a patient.

### BACKGROUND ART

At present, in the field of medicine, genetic testing is employed mainly for diagnosis of infections. By carrying out genetic testing, and taking the observed genetic testing results into account, physicians identify infections to give appropriate treatment.

Future practical application of genetic testing to cancer diagnosis and companion diagnosis has been expected. At present, thanks to rapid development of genetic analysis technologies, next-generation sequencers have been developed, and genome analysis has been accelerated. Genetic testing is now therefore hopeful test means for cancer diagnosis and companion diagnosis. However, genetic testing still requires a lot of time and a high cost for obtaining results.

Examples of the genetic testing methods employed for genetic testing include real-time PCR and real-time LAMP (Loop-Mediated Isothermal Amplification), in which the nucleic acid amplification process is measured in real time. In particular, real-time LAMP has an advantage in its high gene amplification efficiency. At present, genetic testing devices to which genetic testing methods such as real-time PCR and real-time LAMP are applied have become commercially available. Normally, for such a genetic testing device, a pretreatment step for extraction and purification of nucleic acid from samples is carried out by an extraction/purification device provided separately from the test device, or by manual operation of the test device by a skilled operator.

In cases where genetic testing is carried out using a conventional genetic testing device, each of the step of extraction and purification of nucleic acid from samples, the step of injecting a solution containing the nucleic acid to a test chip, and the step of amplification and detection of nucleic acid, requires a complicated operation, so that the operator needs to be highly skilled. Moreover, since the genetic testing devices are expensive, the genetic testing devices have not yet been widely introduced in medical institutions.

Integrated devices which carry out nucleic acid extraction and diagnostic testing for a large number of biological samples have been proposed (for example, Patent Document 1). Such integrated devices are systems for amplifying and detecting nucleic acid contained in extracted samples. The systems are microfluidic systems in which nucleotides are detected by carrying out PCR (polymerase chain reaction) for a large number of nucleotide samples of interest in microfluidic channels (microchannels).

### PRIOR ART DOCUMENT

### [Patent Document]

[Patent Document 1] WO 2009/054870

### SUMMARY OF THE INVENTION

### PROBLEMS TO BE SOLVED BY THE INVENTION

However, the integrated device for nucleic acid extraction and diagnostic testing described in Patent Document 1 or the like is configured such that it operates in association with a complementary rack, and the complementary rack is constituted such that it receives a large number of biological samples in a form suitable for detailed examination and diagnostic analysis, and a large number of holders having various reagents, pipette tips, and containers. The integrated device is a genetic testing device employing a self-contained system having a liquid dispenser for achievement of extraction from nucleic acid and a detection section that are integrated together. That is, since the liquid dispenser of the integrated system does not have a robust structure, nucleic acid contained in samples is influenced by disturbances in the environment in which the genetic testing is carried out.

Moreover, conventional genetic testing devices are large-sized, high-throughput screening type genetic testing devices which are in forms suitable only for genetic testing department of large hospitals, genetic testing companies, and the like. Therefore, since the above genetic testing devices cannot be placed in examination rooms for physicians, those genetic testing devices do not enable genetic testing at a position close to a patient.

In view of this, an object of the present invention is to provide a compact, fully automatic genetic testing device with which a step of extracting and purifying nucleic acid from a sample, a step of injecting a solution containing the nucleic acid to a test chip, and a step of amplifying and detecting the nucleic acid can be fully automatically carried out, and which enables a physician to carry out genetic testing at a position close to a patient while examining the patient. Another object of the present invention is to provide a fully automatic genetic testing device that is applicable to a variety of samples.

### MEANS FOR SOLVING THE PROBLEMS

As a result of intensive study, the present inventors discovered that a compact, fully automatic genetic testing device which enables fully automatic genetic testing, and which enables a physician to carry out the genetic testing at a position close to a patient while examining the patient, can be provided by inclusion of a first unit for extracting and purifying nucleic acid from a sample; a second unit for injecting a solution containing the nucleic acid to a testing chip having a plurality of reaction sites for amplification of the nucleic acid; and a third unit for optically detecting nucleic acid amplified in the reaction site(s); thereby completing the present invention. More specifically, the present invention is constituted by the following technical matters.
(1) A fully automatic genetic testing device comprising: a first unit for extracting and purifying nucleic acid from a sample; a second unit for injecting a solution containing the nucleic acid to a testing chip having a plurality of reaction sites for amplification of the nucleic acid; and a third unit for optically detecting nucleic acid amplified in the reaction sites.
(2) The fully automatic genetic testing device according to (1), wherein the first unit comprises: a pipetting mechanism for transferring a plurality of reagents to be used for extracting and purifying the nucleic acid, between a cartridge containing the reagents and a reaction cell; and a treatment mechanism for pretreatment of the reaction cell; and wherein the treatment mechanism comprises at least one treatment section selected from the group consisting of a heating-cooling treatment section, stirring treatment section, and magnetic field application treatment section.
(3) The fully automatic genetic testing device according to (2), wherein the cartridge comprises: cells with a seal, for individually storing the plurality of reagents; a first cell for storing a piercing tip for opening a hole in the seal; a second cell for storing a pipette tip for transferring the reagents; and a third cell for storing an injection tip for injecting the solution containing the nucleic acid into the reaction sites.
(4) The fully automatic genetic testing device according to (3), wherein the injection tip is constituted by a syringe for retaining the solution containing nucleic acid, and an injection needle, the injection needle being connected to the syringe.
(5) The fully automatic genetic testing device according to (3), wherein the pipetting mechanism comprises: a first mechanism for opening a hole in the seal by bringing the piercing tip into contact with the seal; and a second mechanism for removing the piercing tip from the pipetting mechanism, attaching the pipette tip to the pipetting mechanism, and then transferring the reagents to the reaction cell.
(6) The fully automatic genetic testing device according to (4), wherein the pipetting mechanism comprises: a third mechanism to which the piercing tip is attached; and an injection mechanism comprising, in the third mechanism, the injection tip for injecting the solution containing the nucleic acid from an injection hole of the testing chip to the reaction sites through the injection needle.
(7) The fully automatic genetic testing device according to (6), comprising a fourth mechanism for vibrating the testing chip.
(8) The fully automatic genetic testing device according to (6), comprising a control mechanism for detecting positional information of the tip portion of the injection needle from at least two directions and correcting the position of the tip portion of the injection needle to the position of the injection hole based on the positional information.
(9) The fully automatic genetic testing device according to (6), wherein the testing chip has a plurality of channels connecting the injection hole to the reaction sites; and wherein amplification reaction of the nucleic acid is carried out in the reaction sites.
(10) The fully automatic genetic testing device according to (1), wherein the third unit detects nucleic acid amplified in the reaction sites based on turbidity and/or fluorescence.
(11) The fully automatic genetic testing device according to (3), wherein the cartridge comprises a cartridge base having a nearly circular plate shape, and wherein cells with the seal, the first cell, the second cell, and the third cell are arranged on the circumference of the circle which is the outer periphery of the cartridge base, the device comprising: a drive mechanism capable of moving the pipetting mechanism in the θ direction; and a rotation mechanism capable of rotating the cartridge on the track along which the pipetting mechanism moves.
(12) The fully automatic genetic testing device according to (11), wherein the injection hole of the testing chip is arranged at a position on the track along which the pipetting moves.

### EFFECT OF THE INVENTION

According to the present invention, a fully automatic genetic testing device with which a step of extracting and purifying nucleic acid from a sample, a step of injecting a solution containing the nucleic acid to a test chip, and a step of amplifying and detecting the nucleic acid can be fully automatically carried out, and which enables a physician to carry out genetic testing at a position close to a patient while examining the patient, is provided.

Further, according to the present invention, a fully automatic genetic testing device applicable to a variety of samples, wherein a pretreatment section having a robust structure is employed by avoiding integration of the pretreatment section and the optical test section of the genetic testing device with each other, is provided.

### BRIEF DESCRIPTION OF THE DRAWINGS

Fig. 1 is a perspective view showing an overview of a gene test system D (a master unit and a test unit).
Fig. 2 is a perspective view showing the internal structure of the drawer portion of the fully automatic genetic testing device 1 (test unit) of the gene test system D.
Fig. 3 is a model diagram showing the configuration of the fully automatic genetic testing device 1.
Fig. 4(A) is a perspective view showing the configuration of the cartridge. Fig. 4(B) is a top view of the cartridge. Fig. 4(C) is a cross-sectional view of the cartridge. Fig. 4(D) is a perspective view showing the configuration of the reaction cell.
Fig. 5(A) is a model diagram showing the configuration of the first unit. Fig. 5(B) is an enlarged view of treatment sections in the pretreatment mechanism.
Fig. 6 is a model diagram showing the relationship between the cartridge and the piercing tip.
Fig. 7 is a model diagram showing the relationship between the cartridge and the pipette tip for transferring reagents.
Fig. 8 is a model diagram showing movement of the second mechanism until injection of a solution containing nucleic acid to the injection tip.
Fig. 9 is a flow chart showing each step for extraction and purification of nucleic acid by the first unit.
Fig. 10 is a model diagram showing the configuration of the second unit.
Fig. 11(A) is a top view showing the configuration of the injection tip and the piercing tip. Fig. 11(B) is a cross-sectional view showing the configuration of the injection tip and the piercing tip.
Fig. 12 is a model diagram showing the relationship between the injection needle and the testing chip.
Fig. 13(A) is a front view showing the configuration of the third unit. Fig. 13(B) is a left side view showing the configuration of the third unit.
Fig. 14(A) is a top view showing an overview of a genetic testing device. Fig. 14(B) is a front view showing the overview of the genetic testing device.

### MODE FOR CARRYING OUT THE INVENTION

### <Embodiment 1>

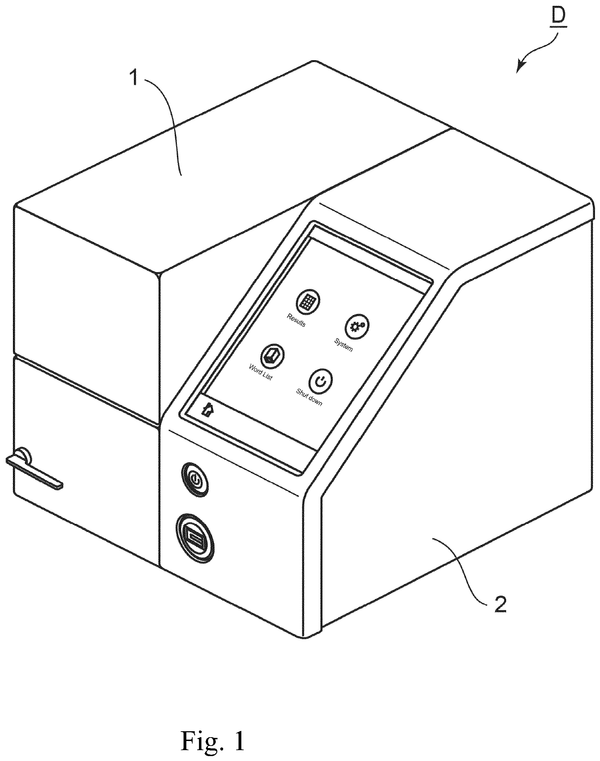

Embodiments of the present invention are described below. Fig. 1 is a perspective view showing an overview of a gene test system D. As shown in Fig. 1, the gene test system D has a master unit 2 and a test unit 1. The master unit 2 and the test unit 1 are electrically connected to each other, and transmission and reception of input data and test results are carried out between the master unit 2 and the test unit 1. In the gene test system D shown in Fig. 1, the master unit 2 and the test unit 1 are separated from each other. However, the type of the system is not limited thereto. For example, a master unit 2 having a control section may be incorporated into a test unit 1 to provide an integrated gene test system D.

The master unit 2 controls the gene test system D. From the master unit 2, turning on/off of the power source, login and logout, sample data, and test items of genetic testing in the gene test system D are input, and measured test results and the like are displayed on a monitor. The user of the gene test system D begins genetic testing by, for example, touching the monitor of the master unit 2. According to commands instructed by the master unit 2, the genetic testing is carried out in the test unit 1.

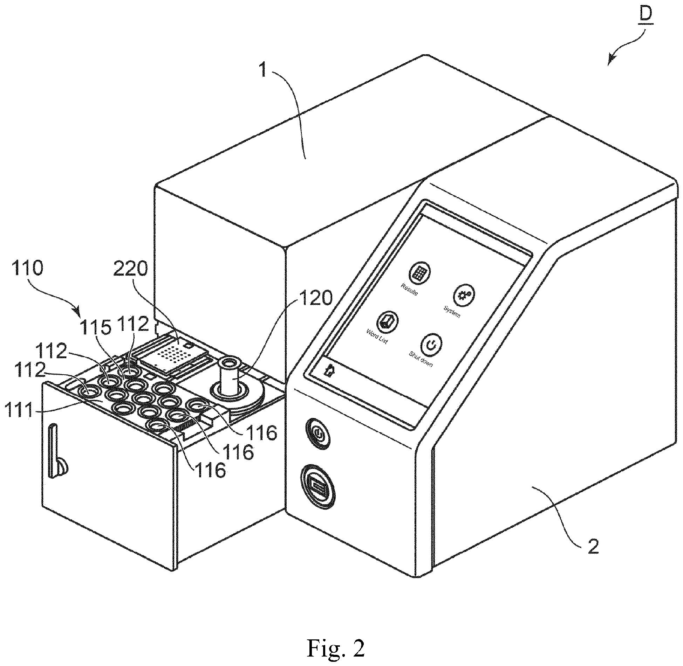

Fig. 2 is a perspective view showing the internal structure of the drawer portion of the test unit 1 of the gene test system D. The test unit 1 has a lever attached in front thereof. In the test unit 1, when the lever is laterally positioned (in a tilted state), the drawer provided in the lower side of the test unit 1 is locked.

In the test unit 1, when the lever is vertically positioned, the drawer provided in the lower side of the test unit 1 is unlocked. The user of the gene test system D can draw the inner portion of the test unit 1 by unlocking the drawer. The user of the gene test system D pulls up the lever of the test unit 1 from the lateral position to the vertical position. By pulling the vertically positioned lever in the direction toward the user of the genetic testing device D, the drawer portion of the test unit 1 can be removed, and samples can then be set therein for performing genetic testing. A technical feature of the gene test system D is the fully automatic genetic testing device 1 as a test unit. The fully automatic genetic testing device 1 of the present invention is described below.

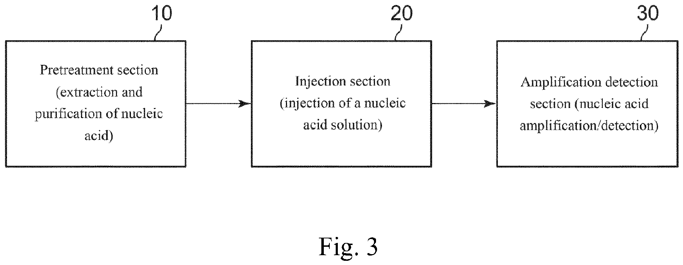

Fig. 3 is a model diagram showing the configuration of the fully automatic genetic testing device 1. As shown in Fig. 3, the fully automatic genetic testing device 1 comprises: a first unit for extracting and purifying nucleic acid from a sample; a second unit for injecting a solution containing the nucleic acid to a testing chip having a plurality of reaction sites for amplification of the nucleic acid; and a third unit for optically detecting nucleic acid amplified in the reaction site(s).

In the fully automatic genetic testing device 1 of the present invention, the first unit corresponds to a pretreatment section 10 for extraction and purification of nucleic acid contained in the sample. The second unit corresponds to an injection section 20 for injection of a solution containing the nucleic acid extracted and purified by the first unit to a test chip. The third unit corresponds to an amplification detection section 30 for optically detecting nucleic acid amplified by the second unit. The fully automatic genetic testing device 1 can carry out fully automatic genetic testing by interactions of the first unit, second unit, and third unit with each other.

### (Configuration of First Unit)

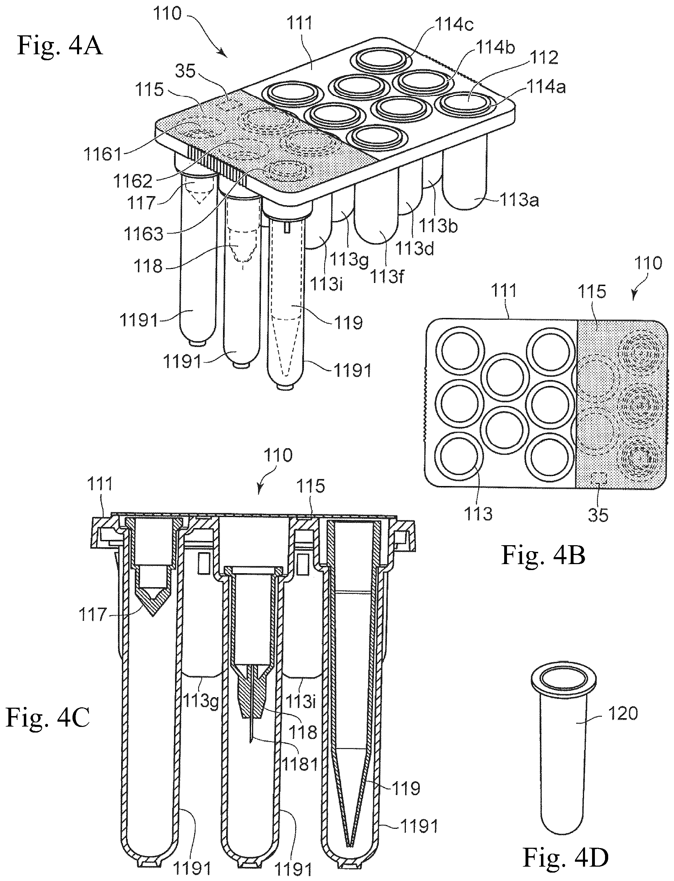

The first unit is a unit for extraction and purification of nucleic acid from the sample, and includes a cartridge 110 containing a plurality of reagents to be used for the extraction and purification of nucleic acid, a reaction cell 120, a pipetting mechanism 130 for transferring the reagents, and a pretreatment mechanism 140 for pretreatment of the reaction cell 120. Fig. 4(A) is a perspective view showing the configuration of the cartridge 110 of the first unit. Fig. 4(B) is a top view of the cartridge 110. Fig. 4(C) is a cross-sectional view of the cartridge 110. Fig. 4(D) is a perspective view showing the configuration of the reaction cell 120 of the first unit.

As shown in Fig. 4(A), the cartridge 110 has, as its basic structure, a cartridge base 111 having a plate-like rectangular shape. To each of a plurality of cartridge base holes 112 formed on the cartridge base 111, a reagent cell with a seal 113a is set. The reagent cells with a seal 113a are cells for separately storing reagents necessary for the extraction and purification of nucleic acid.

The reagent cell with a seal 113a has a cylindrical shape. The reagent cell with a seal 113a has a flange 114a having a circular shape provided in the upper marginal part of the cell. The outer diameter of the flange 114a is larger than the outer diameter of each of the plurality of cartridge base holes 112 formed on the cartridge base 111. Thus, the reagent cell with a seal 113a is fixed to the cartridge base 111 by the flange 114a. The portion in which a reagent is retained in the reagent cell with a seal 113a is positioned in the back side of the cartridge base 111.

The cartridge 110 shown in Fig. 4(A) has 10 reagent cells with a seal 113a to 113i. The number of reagent cells with a seal 113 may be appropriately set depending on the shape and the size of the cartridge base 111. The number of reagent cells with a seal 113 is not limited. The arrangement of the reagent cells with a seal 113 is also not limited, and may be appropriately set depending on the types of the reagents, the concentrations of the reagents, and/or the like. From the viewpoint of operability in the genetic testing, the reagent cells with a seal 113 are preferably arranged in an ordered manner. The cartridge 110 shown in Fig. 4(A) has flanges 114b to 114i for the reagent cells with a seal 113b to 113i, respectively.

A seal 115 for storage of a reagent is attached to the opening section of the reagent cell with a seal 113. The inside of the reagent cell with a seal 113 is tightly closed since the opening section of the reagent cell with a seal 113 is covered by the seal 115. That is, the seal 115 functions as a lid for the reagent cell with a seal 113. The reagent retained in the reagent cell with a seal 113 by the seal 115 is not in contact with the ambient air. Thus, contamination, with impurities, of the reagent retained in the reagent cell with a seal 113 in each step of the genetic testing can be avoided, and reaction of the reagent with oxygen, water, carbon dioxide, and/or the like can also be avoided. The seal 115 shown in Fig. 4(A) covers a surface of the cartridge base 111 which stores two reagent cells with a seal 113, a piercing tip 117, an injection tip 118, and a pipette tip 119. The seal 115 shown in Fig. 4(A) also covers a two-dimensional bar code used for identification of the cartridge base 111. That is, the seal 115 plays a role in hiding the two-dimensional bar code.

The seal 115 tightly adheres to the surface of the cartridge base 111 and the flange 114. The material which can be employed for the seal 115 is required to be capable of tightly adhering to the materials constituting the cartridge base 111 and the flange 114. The material which can be employed as the seal 115 is also required to be capable of adapting to the temperature, pressure, and humidity at which the genetic testing is carried out, and to have an appropriate mechanical strength.

The seal 115 requires sufficient adhesiveness to the cartridge base 111. This is because, in cases where the adhesive strength between the seal 115 and the cartridge base 111 is insufficient, the whole seal 115 is drawn into the reagent cell with a seal 113 upon the later-mentioned contact of the tip portion of the piercing tip 117 to the seal 115.

Specific examples of the seal 115 include thin films composed of a synthetic rubber such as silicone rubber, nitrile rubber, acrylic rubber, or ethylene-propylene rubber, or of a plastic such as a polypropylene, polyethylene, polyethylene terephthalate (PET), polystyrene, or tetrafluoroethylene resin; and metal thin films such as aluminum thin films. The seal 115 may be constituted by a single-layer thin film, or may be a multi-layer thin film constituted by laminating a plurality of single layers composed of the same or different materials. For example, it may be a multi-layer thin film composed of an aluminum layer(s) and a polyethylene terephthalate (PET) layer(s).

The seal 115 has a thickness that allows formation of a hole by penetration of the tip portion of the piercing tip 117. The thickness of the seal 115 is not limited as long as the sealed state of the reagent cell with a seal 113 can be maintained, and as long as a hole can be made by the tip portion of the piercing tip 117.

The seal 115 may be transparent, semitransparent, or opaque. In cases where the seal 115 is transparent or semitransparent, the opening section of the reagent cell with a seal 113 can be externally recognized even after attachment of the seal 115 to the opening section of the reagent cell with a seal 113. The seal 115 attached to the opening section of the reagent cell with a seal 113 may be marked with a symbol, number, color, and/or the like. The cartridge 110 may also be provided with a two-dimensional bar code 35 for identification of the cartridge base 111. In such a case, the seal 115 may be opaque.

As shown in Fig. 4(A), in a tip hole 116 formed on the cartridge base 111, a first cell 1161 is set. The first cell 1161 stores a piercing tip 117 for making a hole on the seal 115 of the reagent cell with a seal 113. The tip portion of the piercing tip 117 has a conical, pointed shape. Thus, by bringing the tip portion of the piercing tip 117 into contact with the surface of the seal 115, and applying pressure thereto, a hole through which the pipette tip 119 can be inserted is formed on the seal 115.

Fig. 4(A) shows a case 1191 for storage of the piercing tip 117, that is, the first cell 1161 may have a case 1191 for storing the piercing tip 117 arranged in the cartridge base 111.

As shown in Fig. 4(A), in another tip hole 116 formed on the cartridge base 111, a second cell 1162 is set. The second cell 1162 stores an injection tip 118 for retaining a solution containing nucleic acid, and for injecting the solution containing nucleic acid to the later-mentioned testing chip 220.

Fig. 4(A) shows a case 1191 for storage of the injection tip 118, that is, the second cell 1162 may have a case 1191 for storing the injection tip 118 arranged in the cartridge base 111.

As shown in Fig. 4(A), in another tip hole 116 formed on the cartridge base 111, a third cell 1163 is set. The third cell 1163 stores a pipette tip 119 for sucking the reagent retained in the reagent cell with a seal 113, and then transferring the reagent to the reaction cell 120. In Fig. 4(A), a case 1191 for storing the pipette tip 119 is shown. That is, the third cell 1163 may have a case 1191 for storing the pipette tip 119. The number of tip holes 116 formed on the cartridge base 111 is not limited, and may be appropriately set, if necessary.

Fig. 4(B) is a top view of the cartridge 110. As shown in Fig. 4(B), the seal 115 covers a surface of the cartridge base 111 which stores two reagent cells with a seal 113, a piercing tip 117, an injection tip 118, and a pipette tip 119. The seal 115 also covers a two-dimensional bar code for identification of the cartridge base 111. Although the seal 115 shown in Fig. 4(B) covers a part of the cartridge base 111, the mode of coverage is not limited thereto. The seal 115 may cover the entire surface of the cartridge base 111. As the seal 115, two or more types of sheets may be employed. Two or more types of sheets may be attached to the respective surfaces of the cartridge base 111 which stores the reagent cells with a seal 113, piercing tip 117, injection tip 118, and pipette tip 119.

Fig. 4(C) is a cross-sectional view of the cartridge 110. As shown in Fig. 4(C), the cartridge 110 has, from left to right, a piercing tip 117, injection tip 118, and pipette tip 119. The arrangement of the piercing tip 117, injection tip 118, and pipette tip 119 is not limited as long as there is no problem in the operation. The cartridge 110 shown in Fig. 4(C) has a case 1191 for storing the piercing tip 117, a case 1191 for storing the injection tip 118, and a case 1191 for storing the pipette tip 119. As shown in Fig. 4(C), the sheet 115 is provided for preventing the piercing tip 117, injection tip 118, and pipette tip 119 from falling off from the cartridge base 111.

The tip of the injection tip 118 is provided with an injection needle 1181 for injection of reagents. The injection needle 1181 is stored in the case 1191. The injection needle 1181 is therefore not in contact with the ambient air.

The reaction cell 120 shown in Fig. 4(D) is a cell to which a sample to be subjected to the genetic testing is introduced. To the reaction cell 120, the reagent retained in the reagent cell with a seal 113 of the cartridge 110 is introduced. In the reaction cell 120, a sample is reacted with the reagent. Thereafter, in the reaction cell 120, nucleic acid contained in the sample is extracted and purified.

The first unit has a pipetting mechanism 130 for transferring the reagent retained in the reagent cell with a seal 113 between the cartridge 110 and the reaction cell 120, and a pretreatment mechanism 140 for pretreatment of the reaction cell 120.

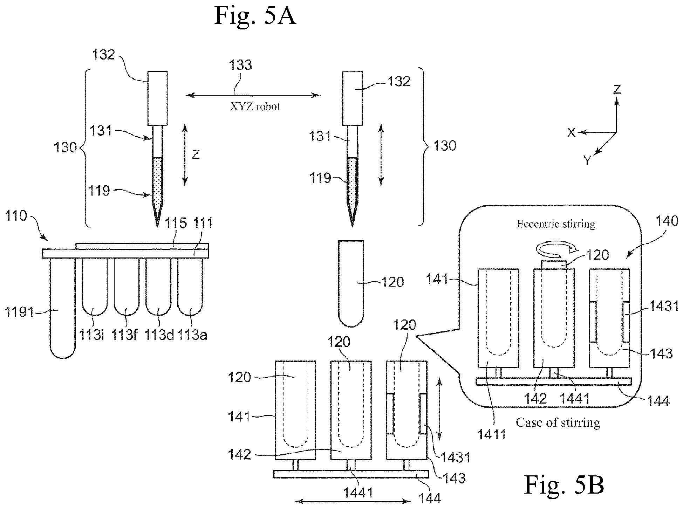

Fig. 5 is a model diagram showing the configuration of a first unit having a pipetting mechanism 130, reaction cell 120, and pretreatment mechanism 140. Fig. 5(A) is an overview of the process of sucking the reagent retained in the reagent cell with a seal 113 of the cartridge 110 by the pipetting mechanism 130 (pipette tip 119), introducing the sucked reagent into the reaction cell 120, and then setting the cell to the pretreatment mechanism 140.

As shown in Fig. 5(A), the pipetting mechanism 130 is constituted by the pipette tip 119, pipette head 131, and driving section 132. The pipette tip 119 is connected to the lower tip portion of the pipette head 131. The pipette head 131 has a protruding portion in the lower tip portion. To the protruding portion, various types of tips such as the pipette tip 119 can be connected. By fitting the pipette tip 119 to the lower tip portion of the pipette head 131, the pipette head 131 can be made to function as the pipetting mechanism 130. That is, the pipette head 131 can have a necessary function by attachment of various tips to the lower tip portion.

The pipette head 131 is connected to the driving section 132. The driving section 132 can freely move on the XY-plane including the plane of the cartridge 110. The driving section 132 can freely move in the Z-axis direction including the vertical direction of the cartridge 110. The driving section 132 can also move on the XY-plane including the opening section of the reaction cell 120. The driving section 132 can move in the Z-axis direction including the vertical direction of the opening section of the reaction cell 120. That is, the driving section 132 can be three-dimensionally driven along the XYZ-axes.

The driving section 132 is controlled by a control section 133. The control section 133 is not limited as long as it is a member capable of moving the driving section 132 to a position at which the operation required for the genetic testing can be carried out. Examples of the control section 133 include XYZ robots and three-dimensional robots.

As shown in Fig. 5(A), the first unit has a pretreatment mechanism 140 for pretreatment of nucleic acid contained in the sample introduced to the reaction cell 120. The pretreatment mechanism 140 has treatment sections for carrying out operations required for extraction and purification of the nucleic acid introduced to the reaction cell 120. As shown in Fig. 5(A), the pretreatment mechanism 140 has a heating-cooling treatment section 141, stirring treatment section 142, and magnetic field application treatment section 143. The pretreatment mechanism 140 has at least one treatment section selected from a heating-cooling treatment section 141, stirring treatment section 142, and magnetic field application treatment section 143. The treatment sections of the pretreatment mechanism 140 can be appropriately set depending on the treatments necessary for the genetic testing.

The heating-cooling treatment section 141 has therein a heater 1411 necessary for heating or cooling of the reaction cell 120. The stirring treatment section 142 is supported by a supporting member 144. The magnetic field application treatment section 143 has therein a magnet 1431 and/or the like necessary for application of magnetic field to the reaction cell 120.

Fig. 5(B) is an enlarged view of the treatment sections. As shown in Fig. 5(B), the reaction cell 120 retaining the nucleic acid and the reagents is inserted into the stirring treatment section 142. The stirring treatment section 142 is connected to a supporting member 144. Rotation of the connection section 1441 of the supporting member 144 causes rotation of the stirring treatment section 142 as well as rotation of the reaction cell 120. For the connection section 1441, a drive motor or the like for generating the torque required for the rotation is employed.

For the stirring treatment section 142, the rotation direction, rotation speed, rotation mode, and/or the like can be set as appropriate. The rotation by the stirring treatment section 142 can also be set to eccentric rotation. For example, by eccentric rotation of the stirring treatment section 142, a stirring effect can be obtained in a length of time about ten times shorter than that in cases of stirring by pipetting, which is conventionally carried out. Further, the eccentric rotation of the stirring treatment section 142 can contribute to shortening of the total process required for the genetic testing.

### (Pretreatment Using First Unit)

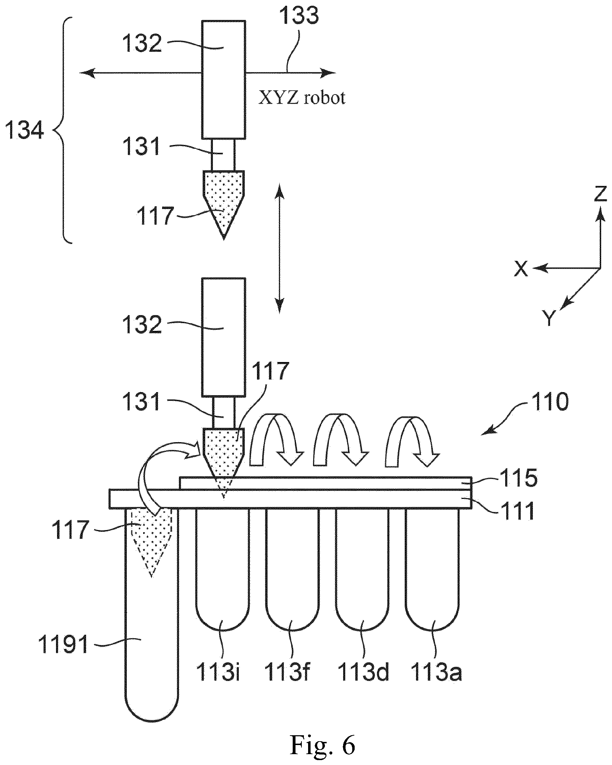

The pretreatment using the first unit is described below. The pretreatment using the first unit means extraction and purification of nucleic acid from a sample. Fig. 6 is a model diagram showing the relationship between the cartridge 110 having a plurality of reagents, and the piercing tip 117.

The pipette head 131 stops at a predetermined position in the cartridge 110 by movement of the driving section 132. The position of the pipette head 131 is set to a position above the tip hole 116 which stores the piercing tip 117 provided in the cartridge 110. The pipette head 131 stops at the corresponding fixed position, on the XY-plane, above the tip hole 116 which stores the piercing tip 117.

The pipette head 131 moves downward in the Z-axis direction. By the downward movement of the pipette head 131 in the Z-axis direction, the tip portion of the pipette head 131 fits into the piercing tip 117. By further applying, in the state where the tip portion of the pipette head 131 is fitted in the piercing tip 117, pressure downward in the Z-axis direction, the tip portion of the pipette head 131 connects to the piercing tip 117. Thus, the pipette head 131 can pick up the piercing tip 117. Here, the pipette head 131 to which the piercing tip 117 is connected is defined as a first mechanism 134.

The first mechanism 134 moves upward in the Z-axis direction. Subsequently, the position of the first mechanism 134 is set to a position above the reagent cell with a seal 113 in which a reagent is retained, provided in the cartridge 110. The first mechanism 134 stops at the corresponding fixed position, on the XY-plane, above the reagent cell with a seal 113.

The first mechanism 134 moves downward in the Z-axis direction. By the downward movement of the first mechanism 134 in the Z-axis direction, the tip portion of the piercing tip 117 of the first mechanism 134 is brought into contact with the seal 115 of the reagent cell with a seal 113. The first mechanism 134 further moves downward in the Z-axis direction. As a result, a predetermined pressure is applied to the seal 115, and the first mechanism 134 creates a hole having a predetermined hole size on the seal 115. The hole created on the seal 115 by the first mechanism 134 has a size sufficient for allowing insertion of the pipette tip 119. As described later, the hole created on the seal 115 is used for suction of the reagent by the pipette tip 119.

After the creation of the hole on the seal 115, the first mechanism 134 returns the piercing tip 117 attached to the pipette head 131 into the tip hole 116 in which the piercing tip 117 had been stored. In cases where a plurality of holes need to be created on the seal 115, the operation of moving the first mechanism 134 is repeatedly carried out. Subsequently, the tip portion of the pipette head 131 is made ready for attachment of the pipette tip 119.

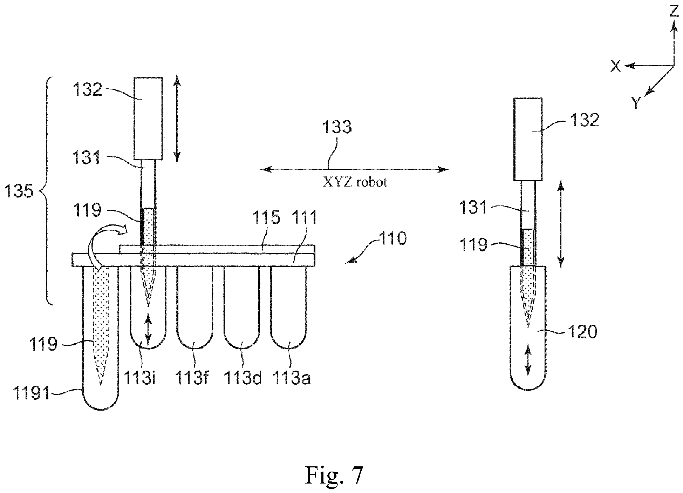

Fig. 7 is a model diagram showing the relationship between the cartridge 110 having a plurality of reagents, and the pipette tip 119 for transferring the reagents. The pipette head 131 stops at the corresponding fixed position, on the XY-plane, above the tip hole 116 which stores the pipette tip 119. By the downward movement of the pipette head 131 in the Z-axis direction, the tip portion of the pipette head 131 connects to the pipette tip 119. The pipette head 131 can pick up the pipette tip 119. Here, the pipette head 131 to which the pipette tip 119 is connected is defined as a second mechanism 135.

The second mechanism 135 moves upward in the Z-axis direction. The second mechanism 135 stops at the corresponding fixed position, on the XY-plane, above the hole formed on the seal 115 of the reagent cell with a seal 113. The second mechanism 135 moves downward in the Z-axis direction. The second mechanism 135 moves downward in the Z-axis direction to pass through the hole, and the tip portion of the pipette tip 119 is brought into contact with the reagent retained in the reagent cell with a seal 113. Subsequently, the pipette tip 119 of the second mechanism 135 sucks the reagent retained in the reagent cell with a seal 113. The pipette tip 119 after the suction of the reagent moves to the reaction cell 120, and the sucked reagent is discharged into the reaction cell 120. In the reaction cell 120, the sample containing nucleic acid reacts with the reagent.

The pipette tip 119 of the second mechanism 135 can suck the solution resulting from the reaction of the solution containing nucleic acid with the reagent, and can then discharge the resulting solution into another reagent cell with a seal 113. The pipette tip 119 of the second mechanism 135 can repeat the process of sucking the reagent retained in the reagent cell with a seal 113, and the process of discharging the reagent into the reaction cell 120. The pipette tip 119 of the second mechanism 135 can also repeat the process of sucking the solution, retained in the reaction cell 120, resulting from the reaction of the sample containing nucleic acid with the reagent, and the process of discharging the resulting solution into another reagent cell with a seal 113. The control section 133 controls movement of the second mechanism 135.

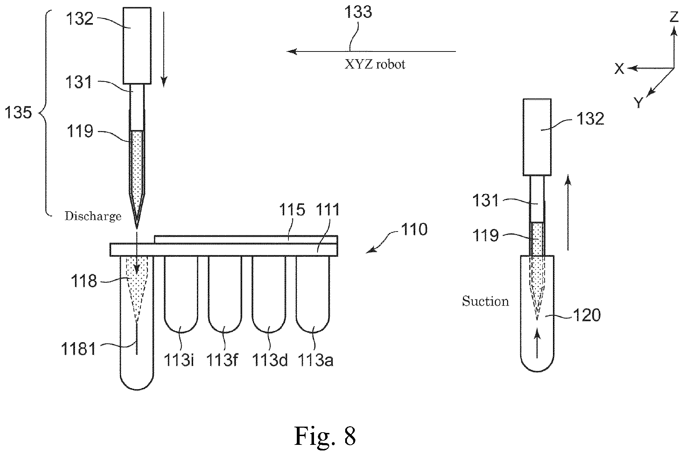

Fig. 8 is a model diagram showing movement of the second mechanism 135 until the injection of the solution containing nucleic acid, obtained by the pretreatment using the first unit, to the injection tip 118.

As shown in Fig. 8, the second mechanism 135 moves between the reaction cell 120 and the reagent cell with a seal 113, and sucks or discharges the reagent retained in the reagent cell with a seal 113, to carry out the operation necessary for the extraction and purification of nucleic acid from the sample. Finally, the reaction cell 120 retains the solution containing nucleic acid after the completion of the process of extraction and purification therein. After the solution containing nucleic acid retained in the reaction cell 120 is sucked by the second mechanism 135, it is discharged into the injection tip 118 provided in the cartridge 110. Thus, in the fully automatic genetic testing device 1 of the present invention, nucleic acid can be extracted and purified from the sample by the first unit, and the operations necessary for the extraction and purification of nucleic acid are fully automatically carried out.

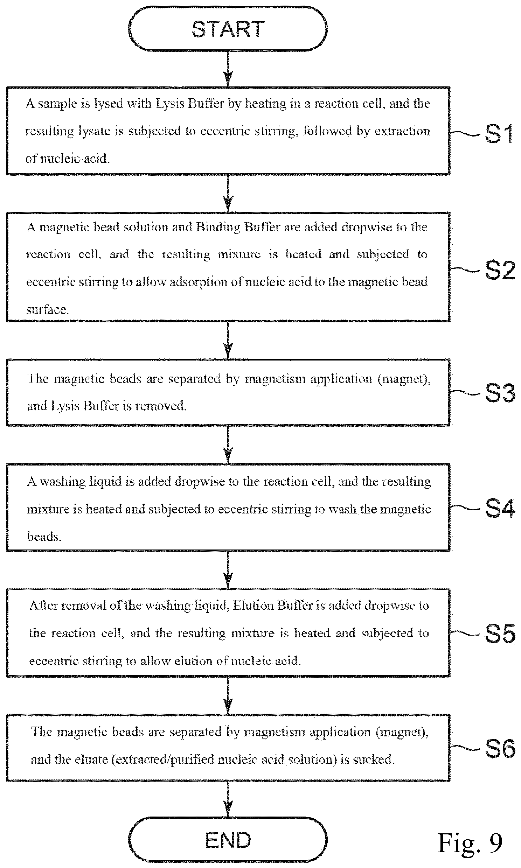

Fig. 9 is a flow chart (protocol) showing the steps of the extraction and purification of nucleic acid using the first unit. First, in the reaction cell 120, a cell lysis buffer such as Lysis Buffer sucked using the pipette tip 119 is added to the sample containing nucleic acid, to lyse cells. The reaction cell 120 is then inserted into the heating-cooling treatment section 141 of the pretreatment mechanism 140, and heated. The heated reaction cell 120 is inserted into the stirring treatment section 142 of the pretreatment mechanism 140, and eccentric stirring is carried out for extraction of nucleic acid contained in the sample (Step 1).

Into the reaction cell 120 that has been processed through Step 1, a magnetic bead solution and a buffer such as Binding Buffer retained in reagent cells with a seal 113 are added dropwise. The reaction cell 120 is then inserted into the heating-cooling treatment section 141 of the pretreatment mechanism 140, and heated. The heated reaction cell 120 is inserted into the stirring treatment section 142 of the pretreatment mechanism 140, and eccentric stirring is carried out to adsorb the extracted nucleic acid to the magnetic beads (Step 2). The first unit may also be capable of carrying out the operations of Step 1 and Step 2 as a single step instead of carrying out the Step 1 and the Step 2 as separate steps. That is, the first unit may have a structure with which Step 1 and Step 2 can be completed by a single step.

The reaction cell 120 that has been processed through Step 2 is inserted into a magnetism application treatment section 143 in the pretreatment mechanism, and magnetism is applied thereto to separate the magnetic beads present in the reaction cell 120. The cell lysis buffer such as Lysis Buffer present in the reaction cell 120 is removed by the pipette tip 119 (Step 3).

Into the reaction cell 120 that has been processed through Step 3, a washing liquid retained in a reagent cell with a seal 113 is added dropwise using the pipette tip 119. The reaction cell 120 containing the washing liquid is then inserted into the heating-cooling treatment section 141 of the pretreatment mechanism, and heated. The heated reaction cell 120 is inserted into the stirring treatment section 142 of the pretreatment mechanism 140, and eccentric stirring is carried out to wash the magnetic beads (Step 4).

The reaction cell 120 passing through step 4 is inserted into the magnetic field application treatment section 143 of the pretreatment mechanism 140 and a magnetic field is applied to separate the magnetic beads existing in the reaction cell 120. The washing liquid present in the reaction cell 120 is removed using the pipette tip 119. Into the reaction cell 120 after the removal of the washing liquid, a buffer such as Elution Buffer is added dropwise. The reaction cell 120 containing the buffer is then inserted into the heating-cooling treatment section 141 of the pretreatment mechanism 140, and heated. The heated reaction cell 120 is inserted into the stirring treatment section 142 of the pretreatment mechanism 140, and eccentric stirring is carried out to elute nucleic acid (Step 5).

The reaction cell 120 that has been processed through Step 5 is inserted into a magnetism application treatment section 143 of the pretreatment mechanism 140, and magnetism is applied thereto to separate the magnetic beads present in the reaction cell 120. By the separation of the magnetic beads, the eluted solution is provided as an extracted/purified solution (Step 6). Thus, according to the protocol shown in Fig. 9, nucleic acid can be extracted and purified from the sample by the first unit of the fully automatic genetic testing device 1.

The solution containing nucleic acid extracted and purified from the sample containing nucleic acid is sucked by the pipette tip 119. The solution containing nucleic acid is then discharged into the injection tip 118 stored in the cartridge 110. Thereafter, the pipette tip 119 is inserted through a tip hole 116 formed in the cartridge 110, and stored in a pipette tip case 1191.

### (Configuration of Second Unit)

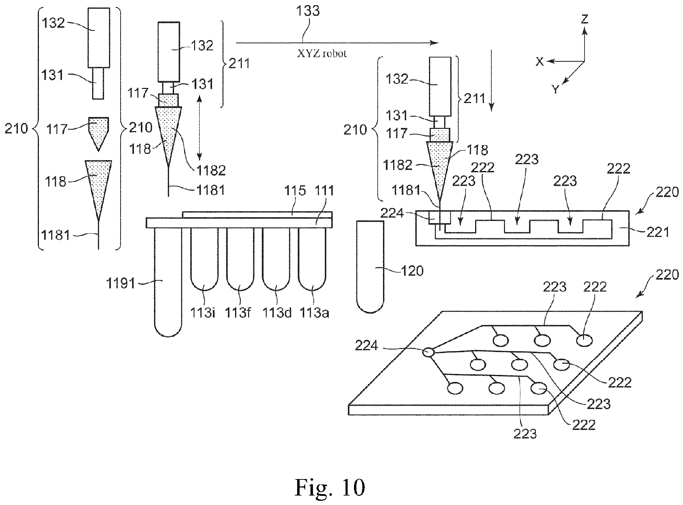

The second unit is a unit for injecting a solution containing nucleic acid to a test chip such as a testing chip having a reaction site for amplification of the nucleic acid obtained using the first unit. The second unit has an injection mechanism 210, and a testing chip 220 having a reaction site for amplification of nucleic acid. Fig. 10 is a schematic diagram showing the configuration of the second unit. As shown in Fig. 10, the injection mechanism 210 of the second unit is constituted by the following three members: pipette head 131, piercing tip 117, and injection tip 118.

The tip portion of the pipette head 131 has a protruding portion, and is connected to a recessed portion of the piercing tip 117. The lower tip portion of the piercing tip 117 has a conical shape, and can fit into the opening section of the injection tip 118. To the lower tip portion of the injection tip 118, an injection needle 1181 is connected. The injection tip 118 has a syringe 1182, which is a portion retaining the solution containing nucleic acid extracted and purified by the first unit, and the injection needle 1181 connected to the syringe 1182.

The second unit also has a testing chip 220 having a plurality of reaction sites for amplification of the nucleic acid extracted and purified using the first unit. The testing chip 220 has a plurality of reaction sites 222 in the inside 221 thereof. The testing chip 220 has, in the inside 221 thereof, microchannels 223 having a role in connecting a reaction site 222 to another reaction site 222. The microchannels 223 are constituted by a plurality of channels for connecting a plurality of reaction sites 222 to each other.

In the testing chip 220, an injection hole 224 is formed. The injection hole 224 functions as a valve for insertion of the injection needle 1181. Through the injection needle 1181, the solution containing nucleic acid retained in the injection tip 118 is injected into the entire testing chip 220 through the injection hole 224.

A member constituting the injection hole 224 is not limited as long as it is a member having a self-sealing property capable of keeping reduced pressure conditions of the inside 221 of the testing chip 220. Specific examples of materials that can be employed for forming the injection hole 224 include silicone resins, acryl silicone resins, polyvinyl chloride resins, and styrene-butadiene copolymer resins.

The testing chip 220 is a closed system, and the pressure of the inside 221 thereof is set lower than the atmospheric pressure to provide reduced pressure. The closed system of the inside 221 of the testing chip 220 can be maintained by the injection hole 224 formed of the member having a self-sealing property. The reaction sites 222 and the microchannels 223 constituting the testing chip 220 are kept under reduced pressure conditions.

In the reaction sites 222 of the testing chip 220, reagents necessary for nucleic acid amplification and nucleic acid detection are preliminarily dried and fixed. Examples of the reagents necessary for nucleic acid amplification and the reagents necessary for nucleic acid detection include, but are not limited to, reagents such as primers, probes, substrates, and enzymes. The necessary reagents are appropriately selected depending on the nucleic acid amplification reaction and the nucleic acid detection reaction carried out in the reaction sites 222.

The nucleic acid amplification reaction to be applied in the reaction sites 222 of the testing chip 220 is not limited. The nucleic acid amplification reaction may be one using the following reactions. More specifically, an example of the nucleic acid amplification reaction is the PCR (Polymerase Chain Reaction) method, in which two kinds of primers are set for a target region, and a target gene is amplified 1,000,000-to 10,000,000-fold while the temperature is changed. Other examples of the LAMP (Loop-mediated isothermal Amplification) method and the SDA (Strand Displacement Amplification) method, which are isothermal amplification methods utilizing chain displacement activity of DNA polymerase. Still other examples of the TMA (Transcription Mediated Amplification) method, the NASBA (Nucleic Acid Sequence Based Amplification) method, and the TRC (Transcription-Reverse transcription Concerted) method, which are amplification methods utilizing T7 RNA polymerase to obtain RNA as the final amplification product.

### (Injection of Solution Containing Nucleic Acid to Testing Chip Using Second Unit)

Injection of the solution containing nucleic acid to the testing chip 220 using the second unit is described below. The injection of the solution containing nucleic acid to the testing chip 220 means injection of the solution containing nucleic acid retained in the injection tip 118 to the injection hole 224.

As shown in Fig. 10, the pipette head 131 picks up again the piercing tip 117 stored in the cartridge 110. Here, the pipette head 131 to which the piercing tip 117 is connected is defined as a third mechanism 211. The tip portion of the piercing tip 117, which is the lower tip portion of the third mechanism 211, connects to the injection tip 118. An injection mechanism 210 is formed by the following three members: the pipette head 131, piercing tip 117, and injection tip 118. Here, in the syringe 1182 of the injection tip 118, the solution containing nucleic acid obtained in the final stage of the pretreatment using the first unit is retained.

The injection mechanism 210 retaining the solution containing nucleic acid stops at the corresponding fixed position, on the XY-plane, above the injection hole 224 of the testing chip 220. By downward movement of the injection mechanism 210 in the Z-axis direction, the injection needle 1181 provided in the tip portion of the injection tip 118 pierces the injection hole 224. At this time, inside of the syringe 1182 of the injection tip 118 is connected to the reaction sites 222 and the microchannels 223 present in the inside 221 of the testing chip 220, through the injection needle 1181.

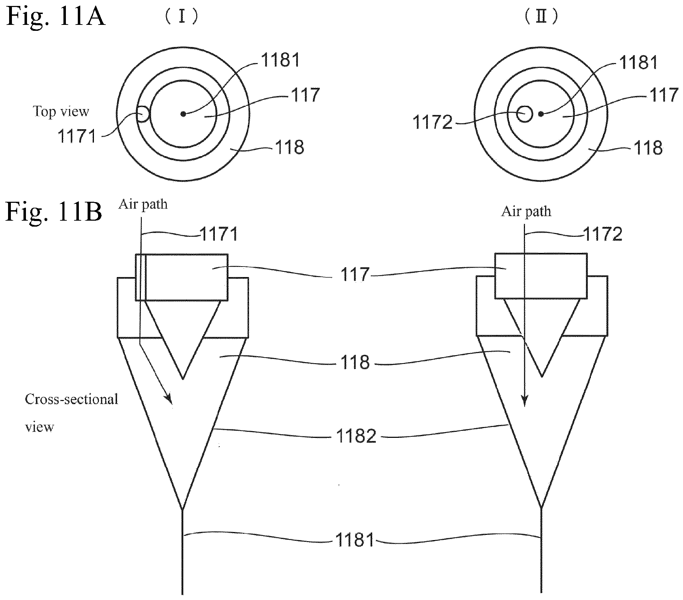

Fig. 11 is a model diagram showing the state where the injection tip 118 is connected to the piercing tip 117. Fig. 11(A) is a top view showing the state where the injection tip 118 is connected to the piercing tip 117. Fig. 11(B) is a cross-sectional view showing the state where the injection tip 118 is connected to the piercing tip 117. Fig. 11 shows two modes in each of which an air path 1171 or 1172 penetrating the piercing tip 117 and the injection tip 118 is formed, wherein these air paths are at different positions.

As shown in Fig. 11, since the injection tip 118 is an open system connected to the ambient air through the air path 1171 or 1172, the atmospheric pressure is applied to the solution containing nucleic acid retained in the syringe 1182 of the injection tip 118. On the other hand, as described above, the pressure in the inside 221 of the testing chip 220 is lower than the atmospheric pressure. Thus, when inside of the syringe 1182 of the injection tip 118 is connected to the inside 221 of the testing chip 220 through the injection needle 1181, the solution containing nucleic acid retained in the syringe 1182 of the injection tip 118 is pushed out by the atmospheric pressure.

The injection mechanism 210 constituting the second unit of the fully automatic genetic testing device 1 of the present invention employs a simple structure in which an air path 1171 or 1172 is formed. Therefore, the injection mechanism 210 is capable of very simply and safely injecting the solution containing nucleic acid into the inside 221 of the testing chip 220 by utilization of the differential pressure between the atmospheric pressure and the pressure of the inside 221 of the testing chip 220. Although the injection mechanism 210 is in a mode in which the air path 1171 or 1172 is formed to apply pressure to the solution containing nucleic acid, the mode capable of applying pressure to the solution containing nucleic acid is not limited thereto. For example, pressure may be applied to the solution containing nucleic acid by the pipette head 131 constituting the third mechanism 211.

In the injection mechanism 210 of the second unit, the pipette head 131 to which the piercing tip 117 is connected forms the tip portion of the third mechanism 211, and an injection tip 118 is provided at the tip portion. In the injection mechanism 210, the presence of the piercing tip 117 between the pipette head 131 and the injection tip 118 is effective for prevention of contamination of the pipette head 131 with the solution containing extracted and purified nucleic acid obtained by the first unit.

Thus, in the second unit, after the injection of the solution containing nucleic acid into the testing chip 220, the injection mechanism 210 returns the injection tip 118 and the piercing tip 117 to their respective tip holes 116 formed in the cartridge 110.

The testing chip 220 may have a fourth mechanism 226 for vibrating the testing chip 220 after the injection of the solution containing nucleic acid to the testing chip 220 by the injection mechanism 210. The vibration by the fourth mechanism may be any of rotation, stopping, and horizontal vibration. The fourth mechanism causes the testing chip 220 to vibrate. The vibration of the testing chip 220 allows sufficient mixing of the reagents such as primers, probe, substrate, and/or enzyme fixed in the reaction sites 222 with the solution containing nucleic acid injected, resulting in improved reactivity in the nucleic acid amplification reaction. The vibration by the fourth mechanism is not limited as long as it can cause the testing chip 220 to vibrate, and may be carried out by employing a motor or the like. The fourth mechanism is attached in the vicinity of the testing chip 220, which is a position where the testing chip 220 can be vibrated.

The fully automatic genetic testing device 1 of the present invention may have a control mechanism 225 for detecting positional information of the tip portion of the injection needle 1181 from at least two directions and correcting the position of the tip portion of the injection needle 1181 to the position of the injection hole 224 based on the positional information.

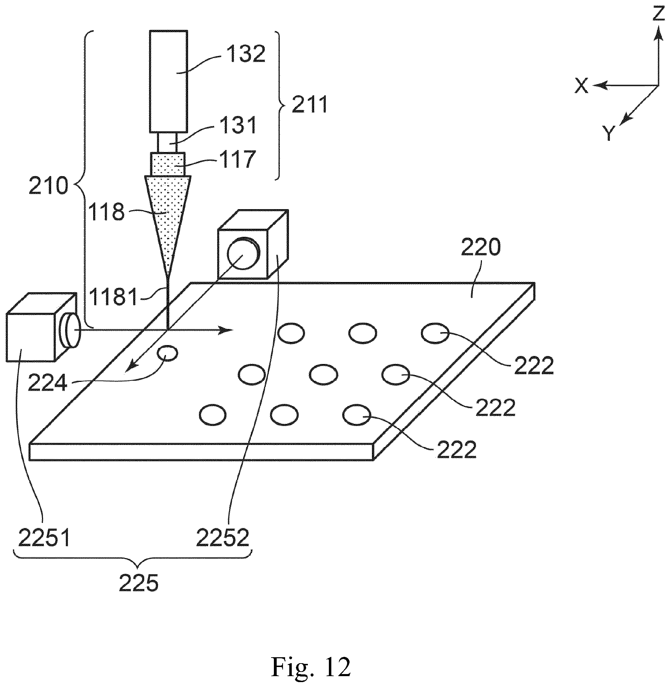

Fig. 12 is a model diagram showing the relationship between the injection needle 1181 and the testing chip 220. As shown in Fig. 12, a camera 2251 and a camera 2252 are arranged on the X-axis and the Y-axis, respectively, of the testing chip 220. The control mechanism 225 is constituted by the camera 2251 and the camera 2252. Positional information 1, on the XY-plane, of the injection needle 1181 can be obtained by the camera 2251 and the camera 2252. The control mechanism 225 preliminarily has positional information 2, on the XY-plane, of the injection hole 224 provided in the testing chip 220. The control mechanism 225 is capable of comparing the obtained positional information 1 with the positional information 2 stored therein, and recognizing deviation of the XY-plane coordinates at which the injection needle 1181 is actually positioned from the XY-plane coordinates preliminarily stored in the control mechanism 225.

The control mechanism 225 recognizes the deviation between the XY-plane coordinates, and controls the position of the injection needle 1181 such that the XY-plane coordinates at which the injection needle 1181 is actually positioned are accurately coincident with the XY-plane coordinates preliminarily stored in the control mechanism.

More specifically, photographs of the position of the injection needle 1181 are taken using the cameras from the two directions. By identifying the coordinates of the injection needle 1181 by image processing, and carrying out feedback control, the position of the needle tip of the injection needle 1181 can be controlled with an accuracy of ±0.1 mm or better.

For example, an injection needle 1181 having an outer diameter of about 0.5 mm may have a slight bend or an error in attachment to the container portion of the injection tip (for example, an injection-molded product of a polypropylene resin), causing a deviation of about ±0.3 mm in terms of the tip position of the injection needle. In cases where the injection hole 224 has a diameter of 1.0 mm, insertion of the needle tip of the injection needle 1181 into the injection hole 224 may be impossible in some cases due to positioning error of the test chip such as the testing chip 220 and deviation of the position of the injection needle 1181.

Thus, in a case where the position of the needle tip of the injection needle 1181 can be controlled with an accuracy of ±0.1 mm or better, and where the positioning accuracy of the test chip such as the testing chip 220 is about ±0.2 mm, the injection needle 1181 can be accurately and sufficiently inserted into the injection hole 224. The detection of the position of the injection needle 1181 is not limited to image processing using cameras, and may be optical detection of the positional information using infrared or the like.

### (Configuration of Third Unit)

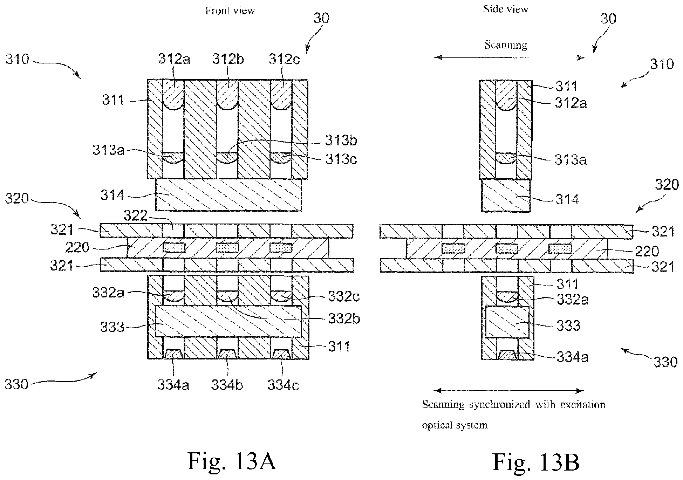

The third unit is a unit for optically detecting the nucleic acid amplified in the plurality of reaction sites 222 of the testing chip 220 of the second unit. Fig. 13(A) is a front view showing the configuration of the third unit. Fig. 13(B) is a left side view showing the configuration of the third unit. As shown in Fig. 13, the third unit is constituted by an excitation optical section 310 arranged in the upper part, a central section 320 having a testing chip 220, and a detection optical section 330 arranged in the lower part.

The excitation optical section 310 includes a base 311 as an outer frame, and an excitation light source, condensing lens 312, and collimating lens 313 arranged in a straight line in the order from the outside. The excitation light source, condensing lens 312, and collimating lens 313 arranged in a straight line form one excitation optical system. The third unit shown in Fig. 13(A) has three optical systems arranged in a line constituted by condensing lenses 312a, 312b, and 312c, respectively, and collimating lenses 313a, 313b, and 313c, respectively. An optical filter 314 is attached below the collimating lenses 313a, 313b, and 313c.

The third unit has the central section 320 for fixing the testing chip 220. The central section 320 is constituted by the testing chip 220, and two heaters 321 for holding the plate-shaped testing chip 220 from both sides. For securing an optical path(s), an aperture(s) may be formed in each heater 321, or a transparent heater prepared by forming a thin film of a metal oxide such as indium tin oxide (ITO) on a glass substrate may be used. As shown in Fig. 13(A), the testing chip 220 has a total of nine reaction sites 222 arranged to form a 3×3 matrix. Each reaction site is arranged in a straight line with one optical system formed in the excitation optical section 310.

The detection optical section 330 contains a base 331 as an outer frame, and a condensing lens 332, optical filter 333, and detector 334 arranged in a straight line in the order from the side of the testing chip 220. The condensing lens 332, optical filter 333, and detector 334 arranged in a straight line form one detection optical system. The third unit shown in Fig. 13(A) has three detection optical systems arranged in a line constituted by condensing lenses 332a, 332b, and 332c, respectively, and detectors 334a, 334b, and 334c, respectively.

### (Optical Detection of Nucleic Acid by Third Unit)

Optical detection of nucleic acid by the third unit is described below. Light generated from the excitation light source in the excitation optical section 310 passes through the condensing lens 312, and then passes through the collimating lens 313. The light that has passed through the collimating lens 313 is filtered by the optical filter 314. The filtered excitation light passes through the holes formed in the upper heater 321, and then passes through the reaction sites 222 of the testing chip 220. The excitation light that has passed through the reaction sites 222 of the testing chip 220 passes through the condensing lenses 332, and is then filtered by the optical filter 333. The excitation light filtered by the optical filter 333 is detected by the detector 334.

As shown in Fig. 13(B), in the optical detection by the third unit, a plurality of optical systems are formed such that they are arranged in a line, and line scanning is carried out. By performing the line scanning while the excitation optical section 310 and the detection optical section 330 are synchronized, all reaction sites 222 of the testing chip 220 can be sequentially optically detected. In the third unit shown in Fig. 13, each of the excitation optical section 310 and the detection optical section 330 has an optical filter suitable for the fluorescent dye for detection of the amplification product. The above optical detection by the third unit is detection using a fluorescent dye, but the optical detection is not limited thereto. For example, the optical detection by the third unit may be detection of an insoluble by-product derived by the nucleic acid amplification reaction, which detection is based on turbidity.

As described above, in the fully automatic genetic testing device 1 of Embodiment 1, a first unit for extracting and purifying nucleic acid from a sample, a second unit for injecting a solution containing the nucleic acid to a testing chip having a plurality of reaction sites for amplification of the nucleic acid, and a third unit for optically detecting nucleic acid amplified in the reaction sites, interact with each other, and a robust structure is employed, so that genetic testing can be fully automatically carried out.

Since each of the first unit, second unit, and third unit of the fully automatic genetic testing device 1 has a compact structure, the whole genetic testing device can be made compact. Therefore, the fully automatic genetic testing device 1 enables genetic testing at a position close to a patient.

### <Embodiment 2>

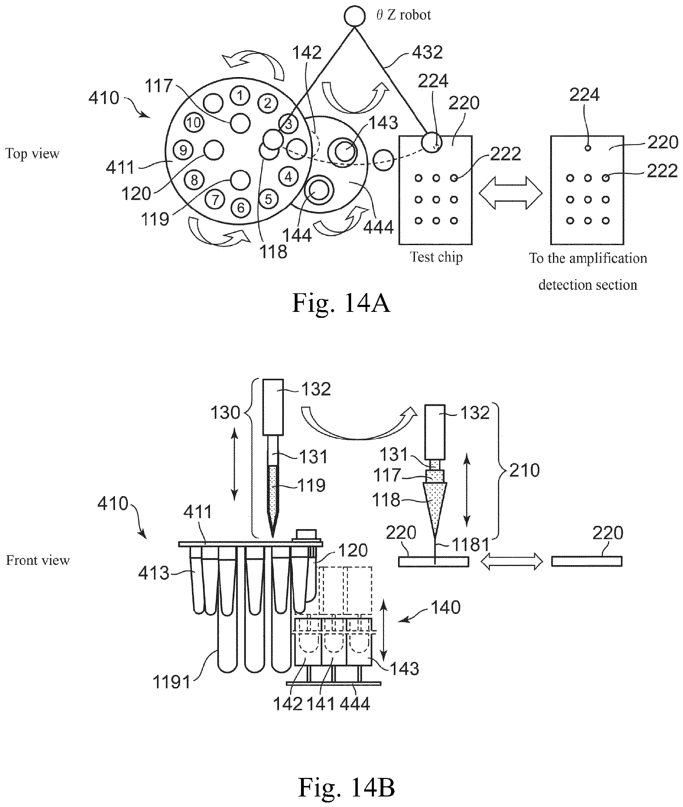

Fig. 14 is a model diagram showing an overview of the fully automatic genetic testing device 1 of Embodiment 2. Fig. 14(A) is a top view showing the overview of the fully automatic genetic testing device 1. Fig. 14(B) is a front view showing the overview of the fully automatic genetic testing device 1. The basic structure of the fully automatic genetic testing device 1 of Embodiment 2 is almost identical to that of the fully automatic genetic testing device of Embodiment 1.

In Fig. 14(A), the fully automatic genetic testing device 1 of Embodiment 2 has a cartridge base 411 having a nearly circular plate shape. Reagent cells with a seal 413 are arranged at regular intervals in a circular pattern along the circumference of the cartridge base 411. In the circle formed by the reagent cells with a seal 413, cells for storing a piercing tip 117, injection tip 118, and pipette tip 119, and a reaction cell 120, are arranged at regular intervals.

As shown in Fig. 14(A), the supporting member 444 supporting the heating-cooling treatment section 141, eccentric stirring treatment section 142, and magnetic field application treatment section 143 constituting the pretreatment mechanism 140 has a nearly circular plate shape. Since the supporting member 444 can rotate, each treatment section of the pretreatment mechanism 140 can also change its position when necessary.

The fully automatic genetic testing device 1 of Embodiment 2 has a driving section 432 which can move the pipetting mechanism 130 in the θ direction, and a rotation mechanism not shown which can rotate the cartridge 410 on the track along which the pipetting mechanism 130 moves. As shown in Fig. 14(A), the θZ driving section 432 capable of moving the pipetting mechanism in the θ direction allows positioning of the pipetting mechanism in the XYZ space and then driving of the pipetting mechanism in the θ direction.

Since the rotation mechanism can rotate the cartridge 410, the reagent cells 413 retaining the plurality of reagents required for the pretreatment, among the reagent cells with a seal 413, can be brought close to the pipetting mechanism 130. By rotating the cartridge 410 and then bringing the plurality of reagents required for the pretreatment close to the pipetting mechanism 130, picking up of the piercing tip 117 and suction of the reagents by the pipette tip 119 can be carried out rapidly and accurately. The θZ driving section 432 moves the pipetting mechanism 130 in the θ direction to carry out the operation of extraction and purification of nucleic acid from the sample on the cartridge base 411 and on the supporting member 444.

As shown in Fig. 14(B), after the completion of the pretreatment by the first unit, the solution containing nucleic acid is injected into the injection tip 118. The injection tip 118 retaining the solution containing nucleic acid pierces the injection hole 224 of the testing chip 220 with the injection needle 1181 to inject the solution containing nucleic acid into the reaction sites 222. The nucleic acid amplified in the reaction sites 222 is detected in an optical detection section 330 of the third unit.

### <Embodiment 3>

The basic structure of the fully automatic genetic testing device 1 of Embodiment 3 is almost identical to that of the fully automatic genetic testing device 1 of Embodiment 1. The fully automatic genetic testing device 1 of Embodiment 3 employs the LAMP (Loop-Mediated Isothermal Amplification) method as the nucleic acid amplification reaction to be applied in the reaction sites 222 of the testing chip 220.

By the employment of the LAMP method for the fully automatic genetic testing device 1, all reactions constituting the nucleic acid amplification reaction can be isothermally carried out; the amplification efficiency in the nucleic acid amplification reaction can be extremely increased; and very high specificity to a particular gene(s) can be achieved.

Further, in cases where the LAMP method is employed for the fully automatic genetic testing device 1, the time required for the nucleic acid amplification reaction can be about one hour. Further, in cases where the LAMP method is employed for the fully automatic genetic testing device 1 with application of a rapid method in which a loop primer and/or the like is added, the time required for the nucleic acid amplification reaction can be about 15 to 30 minutes. Further, in cases where the LAMP method is employed for the fully automatic genetic testing device 1, a large amount of magnesium pyrophosphate is produced as a by-product of the nucleic acid amplification reaction. Since magnesium pyrophosphate is a white insoluble substance, occurrence of the nucleic acid amplification reaction can be visually detected even with the naked eye using as an index the white insoluble substance. Thus, by simple measurement using the third unit, which is capable of optically detecting the level of muddiness (turbidity) caused by the production of the insoluble substance, genetic detection can be carried out.

The PCR method is the nucleic acid amplification reaction that is most widely used at present. However, it needs stepwise control of the reaction temperature for allowing the nucleic acid amplification reaction to proceed. In cases where the PCR method is used, gel electrophoresis needs to be used for checking products produced by the nucleic acid amplification reaction. Further, for checking products produced by the nucleic acid amplification reaction, a detection reaction using a probe needs to be separately carried out. In contrast, the fully automatic genetic testing device 1 of Embodiment 3 enables very simple control of the temperature as well as very simple operations since the device employs the LAMP method.

As important genetic testing devices in the field of medicine, genetic testing devices capable of obtaining the result of genetic testing in 30 minutes after obtaining samples have been demanded. Also for detection of environmental contaminant microorganisms in the environmental field, genetic testing devices capable of obtaining the result of genetic testing immediately after obtaining samples have been demanded. From such a point of view, the fully automatic genetic testing device of Embodiment 3, which employs the LAMP method, is of great technological significance since it has high operability, accuracy, functionality, and rapidity.

Although embodiments of the present invention are described above, the present invention is not limited to the above embodiments, and any modification of conditions or the like which does not depart from the spirit of the present invention is also included within the scope of application of the present invention.

### INDUSTRIAL APPLICABILITY

As described above, the fully automatic genetic testing device of the present invention can fully automatically carry out the process from the step of extracting and purifying nucleic acid contained in a sample to the step of amplifying and detecting the nucleic acid. Therefore, its use as a compact, fully automatic genetic testing device which can be used near a patient can be expected. The fully automatic genetic testing device of the present invention can be used for various industries, especially for the medical device industry, pharmaceutical-related industries, agriculture-related industries, and the like.

### DESCRIPTION OF SYMBOLS

- D: Gene test system
- 1: Fully automatic genetic testing device (test unit)
- 2: Master unit
- 10: Pretreatment section (first unit)
- 20: Injection section (second unit)
- 30: Amplification detection section (third unit)
- 35: Two-dimensional bar code
- 110: Cartridge
- 111: Cartridge base
- 112: Cartridge base hole
- 113: Reagent cell with a seal
- 114: Flange
- 115: Seal
- 116: Tip hole
- 1161: First cell
- 1162: Second cell
- 1163: Third cell
- 117: Piercing tip
- 1171: Air path
- 1172: Air path
- 118: Injection tip
- 1181: Injection needle
- 1182: Syringe
- 119: Pipette tip
- 1191: Pipette tip case
- 120: Reaction cell
- 130: Pipetting mechanism
- 131: Pipette head
- 132: Driving section
- 133: Control section
- 134: First mechanism
- 135: Second mechanism
- 140: Pretreatment mechanism
- 141: Heating-cooling treatment section
- 1411: Heater
- 142: Stirring treatment section
- 143: Magnetic field application treatment section
- 1431: Magnet
- 144: Supporting member
- 1441: Connection section
- 210: Injection mechanism
- 211: Third mechanism
- 220: Testing chip
- 221: Inside of testing chip
- 222: Reaction site
- 223: Microchannel
- 224: Injection hole
- 225: Control mechanism
- 2251: Camera (X-axis direction)
- 2252: Camera (Y-axis direction)
- 310: Excitation optical section
- 311: Base
- 312: Condensing lens
- 313: Collimating lens
- 314: Optical filter
- 320: Central section
- 321: Heater
- 330: Optical detection section
- 331: Base
- 332: Condensing lens
- 333: Optical filter
- 334: Detector
- 410: Cartridge
- 411: Cartridge base (circular)
- 413: Reagent cell with a seal (circular)
- 432: θZ driving section
- 444: Supporting member (circular)

## Claims

1. A fully automatic genetic testing device comprising:
a first unit for extracting and purifying a nucleic acid from a sample;
a second unit for injecting a solution containing the nucleic acid to a testing chip having a plurality of reaction sites for amplification of the nucleic acid; and
a third unit for optically detecting nucleic acid amplified in the reaction sites.

2. The fully automatic genetic testing device according to claim 1,
wherein the first unit comprises:
a pipetting mechanism for transferring a plurality of reagents to be used for extracting and purifying the nucleic acid, between a cartridge containing the reagents and a reaction cell; and
a pretreatment mechanism for pretreatment of the reaction cell;
and
wherein the pretreatment mechanism comprises at least one treatment section selected from the group consisting of a heating-cooling treatment section, stirring treatment section, and magnetic field application treatment section.

3. The fully automatic genetic testing device according to claim 2,
wherein the cartridge comprises:
cells with a seal, for individually storing the reagents;
a first cell for storing a piercing tip for opening a hole in the seal;
a second cell for storing a pipette tip for transferring the reagents; and
a third cell for storing an injection tip for injecting the solution containing the nucleic acid into the reaction sites.

4. The fully automatic genetic testing device according to claim 3, wherein the injection tip is constituted by a syringe for retaining the solution containing the nucleic acid, and an injection needle, the injection needle being connected to the syringe.

5. The fully automatic genetic testing device according to claim3, wherein the pipetting mechanism comprises:
a first mechanism for opening a hole in the seal by bringing the piercing tip into contact with the seal; and
a second mechanism for removing the piercing tip from the pipetting mechanism, attaching the pipette tip to the pipetting mechanism, and then transferring the reagents to the reaction cell.

6. The fully automatic genetic testing device according to claim4, wherein the pipetting mechanism comprises:
a third mechanism to which the piercing tip is attached; and
an injection mechanism comprising, in the third mechanism, the injection tip for injecting the solution containing the nucleic acid from an injection hole of the testing chip to the reaction sites through the injection needle.

7. The fully automatic genetic testing device according to claim 6, comprising a fourth mechanism for vibrating the testing chip.

8. The fully automatic genetic testing device according to claim 6, comprising a control mechanism for detecting positional information of the tip portion of the injection needle from at least two directions and correcting the position of the tip portion of the injection needle to the position of the injection hole based on the positional information.

9. The fully automatic genetic testing device according to claim6,
wherein the testing chip has a plurality of channels connecting the injection hole to a plurality of the reaction sites; and
wherein amplification reaction of the nucleic acid is carried out in the reaction sites.

10. The fully automatic genetic testing device according to claim 1, wherein the third unit detects nucleic acid amplified in the reaction sites based on turbidity and/or fluorescence.

11. The fully automatic genetic testing device according to claim3,
wherein the cartridge comprises a cartridge base having a nearly circular plate shape, and
wherein cells with the seal, the first cell, the second cell, and the third cell are arranged on the circumference of the circle which is the outer periphery of the cartridge base,
the device comprising:
a drive mechanism capable of moving the pipetting mechanism in the θ direction; and
a rotation mechanism capable of rotating the cartridge on the track along which the pipetting mechanism moves.

12. The fully automatic genetic testing device according to claim 11, wherein the injection hole of the testing chip is arranged at a position on the track along which the pipetting mechanism moves.
